(19) Europäisches Patentamt
European Patent Office
Office européen des brevets

(11) Veröffentlichungsnummer: **0 078 768**
**B1**

(12) # EUROPÄISCHE PATENTSCHRIFT

(45) Veröffentlichungstag der Patentschrift: **27.08.86**

(51) Int. Cl.⁴: **C 07 B 37/04**

(21) Anmeldenummer: **82810453.9**

(22) Anmeldetag: **29.10.82**

(54) **Verfahren zur Pd-katalysierten Arylierung von Olefinen mit Arylhalogeniden.**

(30) Priorität: **04.11.81 CH 7055/81**

(43) Veröffentlichungstag der Anmeldung:
**11.05.83 Patentblatt 83/19**

(45) Bekanntmachung des Hinweises auf die Patenterteilung:
**27.08.86 Patentblatt 86/35**

(84) Benannte Vertragsstaaten:
**CH DE FR GB IT LI**

(56) Entgegenhaltungen:
**EP-A-0 040 581**
**DE-A-2 041 563**

**BULLETIN DE LA SOCIETE CHIMIQUE DE FRANCE, September-Oktober 1973, Seiten 2791-2794, 2. Teil, Paris, FR., M. JULIA et al.: "Etude de la condensation de chlorures aromatiques avec les oléfines catalysée par le palladium"**

(73) Patentinhaber: **CIBA-GEIGY AG, Klybeckstrasse 141, CH-4002 Basel (CH)**

(72) Erfinder: **Spencer, Alwyn, Dr., Schützengraben 15, CH-4051 Basel (CH)**

**EP 0 078 768 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Pd-katalysierten Arylierung von Olefinen mit Arylhalogeniden.

Vinylisch oder allylisch substituierte organische Verbindungen, u.a. Styrole und/oder Stilbene, können durch katalytische Umsetzung von entsprechenden Halogeniden mit Olefinen, z.B. Acrylsäuremethylester oder Äthylen, in Gegenwart von tertiären Aminen hergestellt werden. Als Katalysatoren werden bevorzugt Gemische von Palladiumacetat und Triphenylphosphin oder Tri-o-tolylphosphin verwendet. Die Umsetzung kann mit oder ohne Zusatz von organischen Lösungsmitteln, wie Methanol, Acetonitril, N,N-Dimethylformamid, N,N-Dimethylacetamid oder überschüssigem Olefin, vorgenommen werden. Bei der Umsetzung von Halogenbenzolen mit Äthylen unter Druck entstehen je nach Reaktionsbedingungen und/oder Ausgangs-Halogenbenzolen Styrole und/oder Stilbene [vgl. z.B. U.S. Patentschrift 3 922 299 und J. Org. Chem., 43, 2454 und 43, 2941 (1978)]. Gemäss Bull. Chem. Soc. Japan, 46, 1505 (1973) lassen sich verschiedene Olefine, u.a. Äthylen oder Propylen, in Gegenwart von Palladiumschwarz oder $PdCl_2$ und einem Überschuss an Kaliumacetat als Säureakzeptor mit methanolischen Lösungen von Halogenbenzolen, besonders Jodbenzolen, arylieren. Bei diesen vorbekannten Verfahren wird die Palladiumverbindung in einer Menge von mindestens 1 Mol.%, bezogen auf das Halogenbenzol, eingesetzt.

Die Vinylierung von aromatischen Verbindungen ist aus der DE-A-2 041 563 bekannt, wobei die Palladiumverbindung in Mengen von 0,19 bis 32 Mol% eingesetzt wird. Aus dem Bulletin de la Société Chimique de France, September–Oktober 1973, pages 2791–2794 wird die Arylierung von Olefinen mittels Chloraromaten in Gegenwart von Katalysatoren auf Kohle beschrieben. Die verwendete Menge Palladium ist ebenfalls sehr hoch (1,57–1,67 Mol%). Aus der EP-A-0 040 581 ist noch die Arylierung von Olefinen ausgehend aus Aryldicarbonsäurehalogeniden in Gegenwart eines Palladiumkatalysators bekannt.

Gegenstand der Erfindung ist ein neues Verfahren zur Pd-katalysierten Arylierung von Olefinen mit Arylhalogeniden, das bei sehr tiefen Palladiumkonzentrationen und relativ kurzen Reaktionszeiten in guten bis sehr guten Ausbeuten Styrol- und/oder Stilbenderivate liefert.

Nach dem erfindungsgemässen Verfahren können Verbindungen der Formel I

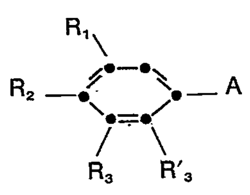 (I),

worin A $C_{2-12}$-Alkenyl, $C_{4-8}$-Cycloalkenyl oder eine Gruppe

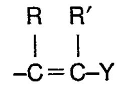

darstellt,

Y $-CN$, $-COR''$, $-COOH$, $-COOR''$, $-CON(R'')_2$ oder

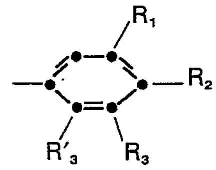,

R Wasserstoff, Methyl, $-CN$ oder $-COOR''$,

R' Wasserstoff, Methyl oder $-CH_2COOR''$,

R'' $C_{1-12}$-Alkyl oder Phenyl und

$R_1$ und $R_3$ unabhängig voneinander Wasserstoff, Phenyl, $C_{1-8}$-Alkyl, $C_{1-5}$-Alkoxy, $-CH(OCH_3)_2$, $-CH(OC_2H_5)_2$,

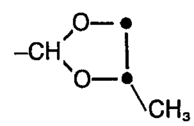, 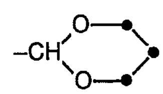, 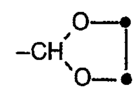,

$-OH$, Cl, F, $-NO_2$, $-CN$, $-CHO$, $-CO-C_{1-4}$-Alkyl, $-COO-C_{1-4}$-Alkyl, $-NHCO-C_{1-4}$-Alkyl, $-NH_2$, $-NH-C_{1-4}$-Alkyl, $-N(C_{1-4}\text{-Alkyl})_2$ oder $-SO^-_3M^+$ bedeuten,

$R_2$ dieselbe Bedeutung wie $R_1$ bzw. $R_3$ haben kann oder eine Gruppe $-CH{=}N$-Phenyl oder $-C(R_4){=}C(R_5)(R_6)$ darstellt und $R'_3$ Wasserstoff, Methyl, $-CN$, $-CHO$ oder $-SO_3C_6H_5$ ist oder $R_1$ und $R_2$ Wasserstoff bedeuten und $R_3$ und $R'_3$ zusammen $-CH{=}CH-CH{=}CH-$ darstellen,

$R_4$ Wasserstoff, $C_{1-4}$-Alkyl, $-CN$ oder $-COO-C_{1-4}$-Alkyl,

$R_5$ Wasserstoff, $C_{1-4}$-Alkyl, $-NHCHO$, $-(CH_2)_m-COO-C_{1-4}$-Alkyl oder $-(CH_2)_m-CN$ mit m = 1 bis 4,

$R_6$ $-CN$ oder $-COO-C_{1-4}$-Alkyl und

$M^+$ ein Metallkation, besonders ein Alkalimetallkation wie $K^+$ oder vor allem $Na^+$ bedeuten, dadurch hergestellt werden, dass man eine Verbindung der Formel II

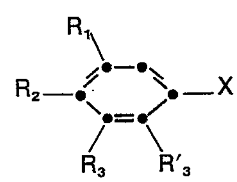 (II)

in Gegenwart eines Alkalimetall- oder Erdalkalimetallsalzes einer aliphatischen Monocarbonsäure mit 1–12 C-Atomen oder der Benzoesäure, eines cyclischen oder N,N-disubstituierten Amids als Lösungsmittel und einer gegebenenfalls arsen- oder phosphorhaltigen Palladium-Verbindung als Katalysator mit einer Verbindung der Formel III

HA (III)

umsetzt, wobei für A, Y, R, R', $R_1$, $R_2$, $R_3$ und $R'_3$ das unter Formel I Angegebene gilt, X Jod oder Brom bedeutet und der Palladiumgehalt 0,1 bis 0,0001 Mol.%, bevorzugt 0,05 bis 0,0001 Mol.%, bezogen auf die Verbindung der Formel II, beträgt. Besonders bevorzugt ist ein Palladiumgehalt von 0,01 bis 0,001 Mol.% und vor allem 0,003 bis 0,008 Mol.%, bezogen auf die Verbindung der Formel II.

Stellt A in Formel I Alkenyl mit 3–12 C-Atomen oder $C_{4-8}$-Cycloalkenyl dar, so kann bei der Reaktion eine Verschiebung der Doppelbindung eintreten, und es entstehen im allgemeinen Isomerengemische.

Bevorzugt stellt mindestens eines von R und R' Wasserstoff dar.

Alkylgruppen R'' und $R_1$ bis $R_5$, Alkoxygruppen $R_1$ bis $R_3$ sowie Alkylgruppen in Substituenten $R_1$ bis $R_6$ können geradkettig oder verzweigt sein. Als Beispiele von definitionsgemässen Gruppen R'' und $R_1$ bis $R_3$ seien erwähnt: Methyl, Äthyl, n-Propyl, Isopropyl, n-Butyl, sek-Butyl, n-Pentyl, 2-Pentyl, n-Hexyl, n-Heptyl, n-Octyl, n-Decyl; Methoxy, Äthoxy, n-Propoxy, Isopropoxy, n-Butoxy, n-Pentyloxy; die Carbonsäuremethyl-, -äthyl-, -n-propyl-, -isopropyl-, -n-butyl- und -sek-butylgruppe; die Acetamid-, Propionamid-, Butyramid- und Valeramidgruppe; die N-Methylamino-, N-Äthylamino-, N-n-Propylamino- und N-n-Butylaminogruppe; die N,N-Dimethylamino-, N,N-Diäthylamino-, N,N-Di-n-propylamino-, N,N-Di-n-butylamino-, N-Methyl-N-äthylamino-, N-Methyl-N-n-propylamino- und N-Äthyl-N-n-butylaminogruppe; $-SO_3^-Na^+$. Als Gruppen $-C(R_4)=C(_5)(R_6)$ kommen bevorzugt solche in Betracht, worin mindestens eines von $R_4$ und $R_5$ Wasserstoff ist, z.B.
$-CH=C(COOCH_3)CH_2COOCH_3$,
$-CH=C(CH_3)COOC_2H_5$,
$-C(COOCH_3)=CHCOOCH_3$,
$COOCH_3$,
$-CH=C(CN)CH_2CH_2CN$, $-CH=CHCOOC_2H_5$
und $-CH=CH-CN$.

Alkylgruppen R'' weisen bevorzugt 1–8 und insbesondere 1–4 C-Atome auf. Besonders bevorzugt bedeutet R'' Methyl, Äthyl oder n-Propyl.

Alkylgruppen $R_1$ bis $R_3$ sind bevorzugt geradkettig und weisen 1–4, besonders 1 oder 2 C-Atome auf. Bevorzugte Alkoxygruppen $R_1$ bis $R_3$ sind die Methoxy- und Äthoxygruppe. Als Alkylgruppen $R_4$ und $R_5$ und Alkylsubstituenten in Gruppen $R_1$ bis $R_6$ sind Methyl und Äthyl bevorzugt.

Stellt Y eine Gruppe 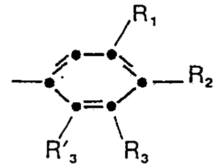

dar, so können deren Substituenten $R_1$, $R_2$, $R_3$ und $R'_3$ dieselben wie die entsprechenden Substituenten an der Verbindung der Formel II oder davon verschieden sein, wobei symmetrische oder asymmetrische Stilbenderivate entstehen. Stilbenderivate können auch dadurch hergestellt werden, dass man als Verbindung der Formel III Äthylen mit gleichen oder verschiedenen Verbindungen der Formel II umsetzt. Im allgemeinen ist die Herstellung von Zimtsäurederivaten bevorzugt.

Besonders bevorzugt verwendet man Verbindungen der Formel II, worin X Brom, $R'_3$ Wasserstoff, $R_1$ und $R_3$ unabhängig voneinander Wasserstoff, Phenyl, $C_{1-3}$-Alkyl, besonders Methyl, Methoxy, $-NO_2$, $-CN$, $-CHO$, Cl, $-NHCO-C_{1-2}$-Alkyl, $-COO-C_{1-2}$-Alkyl, $-CO-C_{1-2}$-Alkyl oder $-N(C_{1-2}$-Alkyl$)_2$ bedeuten und $R_2$ dieselbe Bedeutung wie $R_1$ bzw. $R_3$ haben kann oder eine Gruppe $-C(R_4)=C(R_5)(R_6)$ darstellt, worin $R_4$ Wasserstoff, $-COOCH_3$ oder $-COOC_2H_5$, $R_5$ Wasserstoff, Methyl, Äthyl, $-CH_2COOCH_3$, $-CH_2COOC_2H_5$ oder $-CH_2CH_2CN$ und $R_6$ $-CN$, $-COOCH_3$ oder $-COOC_2H_5$ bedeuten, wobei bevorzugt eines von $R_4$ und $R_5$ Wasserstoff ist.

Besonders bevorzugt verwendet man Verbindungen der Formel II, worin X Brom, $R_1$, $R_3$ und $R'_3$ Wasserstoff und $R_2$ Wasserstoff, Phenyl, Methyl, Methoxy, $-CN$, $-NO_2$, $-CHO$, Cl, $-NHCOCH_3$, $-N(CH_3)_2$ oder eine Gruppe $-CH=CH-R_6$ und $R_6$ $-CN$, $-COOCH_3$ oder $-COOC_2H_5$ darstellen.

Als Verbindungen der Formel III verwendet man vorzugsweise solche, worin A Alkylen mit 2–8 C-Atomen, Cyclopentylen und vor allem Cyclohexy-

R R'
| |
len oder eine Gruppe $-C=C-Y$ darstellt, eines von R und R' Wasserstoff und das andere Methyl oder beide Wasserstoff, und Y Phenyl, $-CN$ oder $-COOR''$ mit R'' = Phenyl oder $C_{1-4}$-Alkyl, besonders Methyl oder Äthyl darstellen. Besonders bevorzugt verwendet man als Verbindung der Formel III Äthylen, Acrylnitril, Acrylsäuremethyl- oder -äthylester.

Bei der Umsetzung mit Äthylen können Styrol- und/oder Stilbenderivate entstehen. Die Reaktion lässt sich hauptsächlich durch Variation des angewendeten Druckes steuern. Stilbene werden vornehmlich bei einem Druck zwischen 0,1 und 1 bar (Normaldruck) gebildet, während bei höherem Druck, zweckmässig einem Druck zwischen 5 und 15 bar, zur Hauptsache Styrolderivate entstehen.

Verbindungen der Formel I worin A eine Gruppe

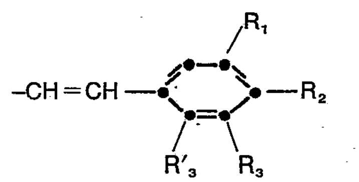

ist, können somit dadurch hergestellt werden, dass man eine Verbindung der Formel II mit Äthylen bei einem Druck zwischen 0,1 und 1 bar umsetzt.

Banz besonders bevorzugt ist die Herstellung von Stilbenderivaten der Formel

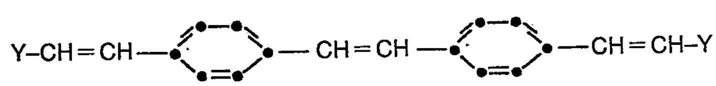

worin Y $-COOC_2H_5$ und insbesondere $-CN$ darstellt, vor allem jedoch des 4-Formylzimtsäurenitrils und des 4,4'-Stilbendialdehyds.

Geeignete Alkalimetall- oder Erdalkalimetallsalze (Basen) für den Einsatz im erfindungsgemässen Verfahren sind z.B. die entsprechenden Natrium-, Kalium-, Lithium-, Barium-, Magnesium- und Calciumsalze, wie die Acetate, Propionate, Butyrate, Laurate und Benzoate. Bevorzugt verwendet man definitionsgemässe Salze, die im Reaktionsmedium mindestens teilweise löslich sind. Besonders bevorzugt sind die Acetate, Propionate und Benzoate, vor allem das Kaliumacetat und ganz besonders das Natriumacetat.

Als cyclische oder N,N-disubstituierte Amide kommen vor allem Verbindungen der Formel IV

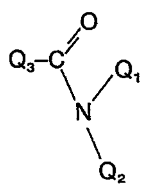

(IV)

in Betracht, worin $Q_1$ und $Q_2$ unabhängig voneinander geradkettiges oder verzweigtes $C_{1-8}$-Alkyl, $C_{5-8}$-Cycloalkyl oder Benzyl oder zusammen $-(CH_2)_3-$, $-(CH_2)_4-$ oder $-(CH_2)_2-O-(CH_2)_2-$ darstellen und $Q_3$ Wasserstoff, geradkettiges oder verzweigtes $C_{1-8}$-Alkyl oder zusammen mit $Q_1$ $-(CH_2)_q-$ mit $q = 3$, 4 oder 5 bedeutet.

Alkylgruppen $Q_1$ und $Q_2$ weisen bevorzugt 1–5 und insbesondere 1–3 C-Atome auf. Stellen $Q_1$ und/oder $Q_2$ Cycloalkylgruppen dar, so handelt es sich insbesondere um Cyclopentyl oder Cyclohexyl. Alkylgruppen $Q_3$ weisen bevorzugt 1 oder 2 C-Atome auf. Als Beispiele von Verbindungen der Formel IV seien genannt: N,N-Dimethylformamid, N,N-Diäthylformamid, N,N-Di-n-butylformamid, N,N-Diisopentylformamid, N,N-Dimethylacetamid, N,N-Dimethylpropionamid, N-Methyl-N-benzylformamid, N-Äthyl-N-cyclohexylformamid, N-Formylpiperidin, N-Formylpyrrolidin, N-Acetylmorpholin, N-Methylpyrrolidon, N-Äthylpyrrolidon und N-Methylpiperidon. Bevorzugt verwendet man als Lösungsmittel N,N-Dimethylformamid, N,N-Diäthylformamid, N,N-Dimethylacetamid oder N-Methylpyrrolidon. Ganz besonders bevorzugt ist N,N-Dimethylformamid. Ferner werden gute Ergebnisse auch in Hexamethylphosphorsäuretriamid als Lösungsmittel erhalten.

Als Katalysatoren können z.B. Palladiumkomplexe der in der U.S. Patentschrift 3 922 299 beschriebenen Art, vor allem Palladium(II)-Komplexe, wie z.B. von $PdCl_2$, $PdBr_2$, $Pd(CN)_2$, $Pd(NO_3)_2$, $Pd(CH_3COCHCOCH_3)_2$, $Pd(OOC-C_{1-12}\text{-Alkyl})_2$, besonders Palladiumacetat, oder auch Palladium(O)-Komplexe, wie z.B. Komplexe von Bis-(Dibenzylidenaceton)palladium(O) und Bis-(Phenylisonitril)palladium(O), mit 3-wertigen Phosphor- oder Arsenverbindungen, wie Trialkyl-, Triaryl-, Trialkoxy- und Triphenoxyphosphinen oder -arsinen oder gemischt substituierten dreiwertigen Phosphor- oder Arsenverbindungen, verwendet werden. Als Beispiele solcher Phosphor- oder Arsenverbindungen seien genannt: Triphenylarsin, Diphenylmethylphosphin, Diphenylmethoxyphosphin, Trimethylphosphin, Triäthylphosphin, Tri-n-butylphosphin, Triphenylphosphin, Phenyl-di-n-butoxyphosphin, Tri-o-tolylphosphin und Triphenylphosphit. Die genannten Komplexe können als solche eingesetzt oder in situ, d.h. im Reaktionsmedium, gebildet werden.

Der Phosphor- bzw. Arsenligand wird zweckmässig in 2–10-fachem molarem Überschuss, bezogen auf das Palladium, verwendet. Im Falle von Verbindungen der Formel II mit stark elektronenanziehenden Substituenten, wie $-NO_2$, $-CHO$ oder $-CN$, kann auch ohne Phosphor- oder Arsenligand gearbeitet werden. Stellt X in Formel II Jod dar, so wird im allgemeinen kein Ligand mitverwendet. Bevorzugt verwendet man als Katalysatoren Gemische aus $PdCl_2$, Palladiumacetat oder Bis-(Dibenzylidenaceton)palladium(O) und Tri-n-butylphosphin, Triphenylphosphin, Tri-o-tolylphosphin oder Triphenylphosphit. Besonders bevorzugt sind Gemische aus Palladiumacetat und Triphenylphosphin oder Tri-o-tolylphosphin und ganz besonders das Diacetato-bis-(tris-o-tolylphosphin)-palladium(II).

Die Reaktionstemperaturen für die erfindungsgemässe Umsetzung liegen zweckmässig zwischen 50 und 200°C, vorzugsweise zwischen 100 und 150°C.

Hohe Ausbeuten bei niedrigen Katalysatorkonzentrationen erzielt man insbesondere dann, wenn man das erfindungsgemässe Verfahren in N,N-Dimethylformamid als Lösungsmittel unter Verwendung von Kalium- oder Natriumacetat als Base und einem Gemisch aus Palladiumacetat und Triphenylphosphin oder Tri-o-tolylphosphin und vor allem Diacetato-bis-(tris-o-tolylphosphin)-palladium(II) als Katalysator durchführt.

Die erfindungsgemäss herstellbaren Verbindungen und deren Verwendungen sind zum grossen Teil bekannt. Sie können beispielsweise direkt als optische Aufheller oder aber als Zwischenprodukte für optische Aufheller eingesetzt werden. Derartige optische Aufheller sind z.B. in der U.S. Patentschrift 4.108.887 beschrieben. Die erfindungsgemäss hergestellten Verbindungen können ferner in an sich bekannter Weise, gegebenenfalls unter Einführung von geeigneten funktionellen Gruppen, wie Aminogruppen, und/oder durch Sulfonierung der aromatischen Reste in Farbstoffe oder optische Aufheller übergeführt werden [vgl. z.B. Encyclopedia of Chemical Technology. 2. Auflage, Bd. 19, S. 1–16]. Stilbene und Stilbenderivate finden auch Anwendung als Scintillatoren, Klebstoffadditive, Insektizide oder Lichtstabilisatoren; vgl. z.B. Chemical Abstracts, 78, 39 352; 84, 137 386 und 85, 22 416. Styrole und Styrolderivate eignen sich auch zur Herstellung von Homo- und Copolymeren.

In den folgenden Beispielen bezieht sich der Pd-Gehalt auf die jeweilige Verbindung der Formel II.

$$\text{Umsatzzahl} = \frac{\text{Mol Endprodukt}}{\text{Mol Pd-Verbindung.}}$$

Beispiel 1:

Unter Argon wird eine Stammlösung von 0,0561 g ($2{,}5 \times 10^{-4}$ Mol) Palladiumacetat und 0,304 g ($10^{-3}$ Mol) Tri-o-tolyl-phosphin in 100 ml N,N-Dimethylformamid (DMF) hergestellt. Dann werden unter Argon zu 9 ml DMF 1 ml Stammlösung [enthaltend 0,000561 g ($2{,}5 \times 10^{-6}$ Mol) Palladiumacetat, Pd-Gehalt = 0,01 Mol.%, und 0,00304 g ($10^{-5}$ Mol) Tri-o-tolylphosphin], 4,63 g (25 mMol) 4-Brombenzaldehyd, 2,08 ml (31,25 mMol) Acrylnitril und 2,56 g (31,25 mMol) wasserfreies Natriumacetat gegeben, und das Gemisch wird während 6 Std. bei 120 °C gerührt. Danach wird das Gemisch in 100 ml Wasser eingegossen, mit 2 × 25 ml Dichlormethan extrahiert, und der Extrakt wird mit 5 g Magnesiumsulfat während 15 Minuten getrocknet. Nach dem Entfernen des Dichlormethans und etwas DMF am Rotationsverdampfer wird das Rohprodukt im Vakuum destilliert. Man erhält 3,2 g (20 mMol) 4-Formylzimtsäurenitril als fast weissen Feststoff; Ausbeute 81% d.Th. (Umsatzzahl 8100); Fp. 92–98 °C; Sdp. 157–163 °C/133 Pa. Analyse für $C_{10}H_7NO$ (Molgewicht 157,17): berechnet C 76, H 4,49 N 8,91, gefunden C 76,35 H 4,60 N 9,01. Das Produkt ist ein Isomerengemisch bestehend aus ca. 77% trans- und 23% cis-Isomer.

Beispiel 2:

Es wird wie in Beispiel 1 beschrieben vorgegangen, jedoch mit einer Reaktionszeit von 4 Std. bei 130 °C. Man erhält 3,22 g (20,5 mMol) 4-Formylzimtsäurenitril, entsprechend einer Ausbeute von 82% d.Th. (Umsatzzahl 8150; Pd-Gehalt 0,01 Mol.%).

Beispiel 3:

Es wird wie in Beispiel 1 beschrieben vorgegangen, jedoch mit einer Reaktionszeit von 2,5 Std. bei Rückflusstemperatur. Man erhält 3,30 g (21 mMol) 4-Formylzimtsäurenitril, entsprechend einer Ausbeute von 84% d.Th. (Umsatzzahl 8400; Pd-Gehalt 0,01 Mol.%).

Beispiel 4:

Es wird wie in Beispiel 1 beschrieben vorgegangen, jedoch unter Verwendung einer Stammlösung, die 0,262 g ($10^{-5}$ Mol) Triphenylphosphin anstelle von Tri-o-tolylphosphin enthält. Nach einer Reaktionszeit von 9 Std. bei 130 °C erhält man 3,30 g (21 mMol) 4-Formylzimtsäurenitril, entsprechend einer Ausbeute von 84% d.Th. (Umsatzzahl 8400; Pd-Gehalt 0,01 Mol.%).

Beispiel 5:

Es wird wie in Beispiel 1 beschrieben vorgegangen, jedoch unter Verwendung einer Stammlösung, die 0,246 ml ($10^{-5}$ Mol) Tri-n-butylphosphin anstelle von Tri-o-tolylphosphin enthält. Nach einer Reaktionszeit von 4 Std. bei 130 °C erhält man 3,4 g (21,7 mMol) 4-Formylzimtsäurenitril, entsprechend einer Ausbeute von 87% d.Th. (Umsatzzahl 8700; Pd-Gehalt 0,01 Mol.%).

Beispiel 6:

Es wird wie in Beispiel 1 beschrieben vorgegangen, jedoch unter Verwendung einer Stammlösung, die 0,263 ml ($10^{-5}$ Mol) Triphenylphosphit enthält. Nach einer Reaktionszeit von 6,5 Std. bei 130 °C erhält man 3,35 g (21,3 mMol) 4-Formylzimtsäurenitril, entsprechend einer Ausbeute von 85% d.Th. (Umsatzzahl 8500; Pd-Gehalt 0,01 Mol.%).

Beispiel 7:

Es wird wie in Beispiel 1 beschrieben vorgegangen, jedoch unter Verwendung einer Stammlösung, die kein Phosphin enthält. Nach einer Reaktionszeit von 10 Std. bei 130 °C erhält man 3,06 g (19,5 mMol) 4-Formylzimtsäurenitril, entsprechend einer Ausbeute von 78% d.Th. (Umsatzzahl 7800; Pd-Gehalt 0,01 Mol.%).

Beispiel 8:

Es wird wie in Beispiel 1 beschrieben vorgegangen, jedoch unter Verwendung einer Stammlösung, die 0,152 g ($5 \times 10^{-6}$ Mol) Tri-o-tolylphosphin enthält. Nach einer Reaktionszeit von 4 Std. bei 130 °C erhält man 3,51 g (22,36 mMol) 4-Formylzimtsäurenitril entsprechend einer Ausbeute von 89% d.Th. (Umsatzzahl 8900; Pd-Gehalt 0,01 Mol.%).

Beispiel 9:

Unter Argon wird eine Stammlösung aus 0,0520 g ($6{,}25 \times 10^{-5}$ Mol) Diacetato-bis-(tris-o-tolylphosphin)palladium(II) in 25 ml DMF hergestellt. Zu 9 ml DMF werden unter Argon 1 ml Stammlösung, 4,63 g (25 mMol) 4-Brombenzaldehyd, 2,08 ml (31,25 mMol) Acrylnitril und 2,26 g (27,5 mMol) wasserfreies Natriumacetat gegeben, und das Reaktionsgemisch wird während 4 Std. bei 130 °C gerührt. Nach der Aufarbeitung gemäss Beispiel 1 erhält man 3,50 g (22,3 mMol) 4-Formylzimtsäurenitril, entsprechend einer Ausbeute von 89% d.Th. (Umsatzzahl 8900; Pd-Gehalt 0,01 Mol.%.

Beispiel 10:

Es wird wie in Beispiel 9 beschrieben vorgegangen, jedoch unter Verwendung von 1,83 ml (27,5 mMol) Acrylnitril. Man erhält 3,44 g (21,9 mMol) 4-Formylzimtsäurenitril, entsprechend einer Ausbeute von 88% d.Th. (Umsatzzahl 8800; Pd-Gehalt 0,01 Mol.%).

Beispiel 11:

Es wird wie in Beispiel 1 beschrieben vorgegangen, jedoch unter Verwendung von 4 ml anstatt

9 ml DMF. Nach einer Reaktionszeit von 4 Std. bei Rückflusstemperatur erhält man 3,11 g (19,8 mMol) 4-Formylzimtsäurenitril, entsprechend einer Ausbeute von 79% d.Th. (Umsatzzahl 7900; Pd-Gehalt 0,01 Mol.%).

Beispiel 12:

Es wird wie in Beispiel 1 beschrieben vorgegangen, jedoch unter Verwendung von N-Methylpyrrolidon als Lösungsmittel, und zwar sowohl für die Stammlösung als auch für das Reaktionsgemisch. Nach einer Reaktionszeit von 6 Std. bei 130 °C erhält man 1,96 g (12,5 mMol) 4-Formylzimtsäurenitril, entsprechend einer Ausbeute von 50% d.Th. (Umsatzzahl 5000; Pd-Gehalt 0,01 Mol.%).

Beispiel 13:

Es wird wie in Beispiel 12 beschrieben vorgegangen, jedoch unter Verwendung von N,N-Dimethylacetamid (DMA) als Lösungsmittel, und zwar sowohl für die Stammlösung als auch für das Reaktionsgemisch. Nach einer Reaktionszeit von 8 Std. bei 130 °C erhält man 2,28 g (14,5 mMol) 4-Formylzimtsäurenitril, entsprechend einer Ausbeute von 58% d.Th. (Umsatzzahl 5800; Pd-Gehalt 0,01 Mol.%).

Beispiel 14:

Unter Argon wird eine Stammlösung aus 0,0260 g ($3,125 \times 10^{-5}$ Mol) Diacetato-bis-(tris-o-tolylphosphin)palladium(II) in 25 ml DMF hergestellt. Zu 4 ml DMF werden unter Argon 1 ml Stammlösung, 4,63 g (25 mMol) 4-Brombenzaldehyd, 1,83 ml (27,5 mMol) Acrylnitril und 2,26 g (27,5 mMol) wasserfreies Natriumacetat gegeben, und das Gemisch wird 9 Std. bei 130 °C gerührt. Nach der Aufarbeitung gemäss Beispiel 1 erhält man 3,25 g (20,7 mMol) 4-Formylzimtsäurenitril, entsprechend einer Ausbeute von 83% d.Th. (Umsatzzahl 16 600; Pd-Gehalt 0,005 Mol.%.

Beispiel 15:

Es wird wie in Beispiel 14 beschrieben vorgegangen, jedoch unter Verwendung von 9 ml DMF. Nach einer Reaktionszeit von 6,5 Std. erhält man 3,37 g (21,5 mMol) 4-Formylzimtsäurenitril, entsprechend einer Ausbeute von 86% d.Th. (Umsatzzahl 17 200; Pd-Gehalt 0,005 Mol.%).

Beispiel 16:

Es wird wie in Beispiel 14 beschrieben vorgegangen, jedoch mit einer Stammlösung aus 0,0281 g ($1,25 \times 10^{-4}$ Mol) Palladiumacetat und 0,066 ml ($2,5 \times 10^{-4}$ Mol) Triphenylphosphit in 100 ml DMF. Nach einer Reaktionszeit von 7 Std. erhält man 2,57 g (16,4 mMol) 4-Formylzimtsäurenitril, entsprechend einer Ausbeute von 65% d.Th. (Umsatzzahl 13 000; Pd-Gehalt 0,005 Mol.%).

Beispiel 17:

Es wird wie in Beispiel 14 beschrieben vorgegangen, jedoch mit einer Stammlösung aus 0,0281 g ($1,25 \times 10^{-4}$ Mol) Palladiumacetat und 0,0615 g ($2,5 \times 10^{-4}$ Mol) Tri-n-butylphosphin in 100 ml DMF. Nach einer Reaktionszeit von 7 Std. erhält man 2,84 g (18,1 mMol) 4-Formylzimtsäurenitril, entsprechend einer Ausbeute von 72% d.Th. (Umsatzzahl 14 400; Pd-Gehalt 0,005 Mol.%).

Beispiel 18:

Zu 100 ml DMF werden unter Argon 46,25 g (250 mMol) 4-Brombenzaldehyd, 18,31 ml (275 mMol) Acrylnitril, 22,55 g (275 mMol) wasserfreies Natriumacetat und 0,0104 g ($1,25 \times 10^{-5}$ Mol) Diacetato-bis-(tris-o-tolylphosphin)palladium(II) gegeben, und das Reaktionsgemisch wird während 8 Std. bei 130 °C gerührt. Nach der Aufarbeitung gemäss Beispiel 1 erhält man 34,83 g (222 mMol) 4-Formylzimtsäurenitril, entsprechend einer Ausbeute von 89% d.Th. (Umsatzzahl 17 800; Pd-Gehalt 0,005 Mol.%).

Beispiel 19:

Es wird wie in Beispiel 10 beschrieben vorgegangen, jedoch unter Verwendung von 1,81 g (27,5 mMol) wasserfreiem Lithiumacetat. Das Reaktionsgemisch wird unter Rückfluss gerührt, bis eine Temperatur von 130 °C erreicht ist, und danach bei 130 °C gehalten. Nach einer Reaktionszeit von 8 Std. erhält man 2,91 g (18,5 mMol) 4-Formylzimtsäurenitril, entsprechend einer Ausbeute von 74% d.Th. (Umsatzzahl 7400; Pd-Gehalt 0,01 Mol.%).

Beispiel 20:

Unter Argon wird eine Stammlösung aus 0,0260 g ($3,125 \times 10^{-5}$ Mol) Diacetato-bis-(tris-o-tolylphosphin)palladium(II) in 50 ml DMF hergestellt. Zu 9 ml DMF werden unter Argon 1 ml Stammlösung, 4,63 g (25 mMol) 4-Brombenzaldehyd, 1,83 ml (27,5 mMol) Acrylnitril, 2,26 g (27,5 mMol) wasserfreies Natriumacetat gegeben, und das Reaktionsgemisch wird 10 Std. bei 130 °C gerührt. Man erhält 2,86 g (18,2 mMol) 4-Formylzimtsäurenitril, entsprechend einer Ausbeute von 73% d.Th. (Umsatzzahl 29 200; Pd-Gehalt 0,0025 Mol.%).

Beispiel 21:

Unter Argon wird eine Stammlösung aus 0,0208 g ($2,5 \times 10^{-5}$ Mol) Diacetato-bis-(tris-o-tolylphosphin)palladium(II) in 25 ml DMF hergestellt. Dann wird die in Beispiel 20 beschrieben vorgegangen, unter Verwendung von 0,25 ml Stammlösung. Nach einer Reaktionszeit von 24 Std. bei 130 °C erhält man 3,1 g (19,7 mMol) 4-Formylzimtsäurenitril, entsprechend einer Ausbeute von 79% d.Th. (Umsatzzahl 79 000; Pd-Gehalt 0,001 Mol.%).

Beispiel 22:

Unter Argon wird eine Stammlösung aus 0,208 g ($2,5 \times 10^{-4}$ Mol) Diacetato-bis-(tris-o-tolylphosphin)palladium(II) in 100 ml DMF hergestellt. Zu 9 ml DMF werden unter Argon 1 ml Stammlösung, 5,05 g (25 mMol) 4-Bromnitrobenzol, 1,83 ml (27,5 mMol) Acrylnitril und 2,2 g (27,5 mMol) wasserfreies Natriumacetat gegeben, und das Reaktionsgemisch wird 4 Std. bei 130 °C gerührt. Nach der Aufarbeitung gemäss Beispiel 1 und dem Umkristallisieren des Rohproduktes aus Toluol/Tetrachlorkohlenstoff erhält man 3,8 g (21,8 mMol) 4-Nitrozimtsäurenitril als gelbe Kristalle vom Fp. 202 °C in einer Ausbeute von 87% d.Th. (Umsatzzahl 8700; Pd-Gehalt 0,01 Mol.%). Analyse für $C_9H_6N_2O_2$ (Molgewicht 174,16): berechnet C 62,07 H 3,47 N 16,09, gefunden C 62,36 H 3,51 N 16,10.

Beispiel 23:

Unter Argon wird eine Stammlösung aus 0,0560 g ($2,5 \times 10^{-4}$ Mol) Palladiumacetat und 0,262 g ($10^{-3}$ Mol) Triphenylphosphin in 100 ml DMF hergestellt. Zu 9 ml DMF werden unter Argon 1 ml Stammmlösung, 2,61 ml (25 mMol) Brombenzol, 2,99 ml (27,5 mMol) Acrylsäureäthylester und 2,26 g (27,5 mMol) Natriumacetat gegeben, und das Reaktionsgemisch wird 8 Std. bei 130 °C gerührt. Nach der Aufarbeitung gemäss Beispiel 1 erhält man 2,86 g (16,25 mMol) Zimtsäureäthylester, entsprechend einer Ausbeute von 65% d.Th. (Umsatzzahl 6500; Pd-Gehalt 0,01 Mol.%).

Beispiel 24:

Es wird wie in Beispiel 23 beschrieben vorgegangen unter Verwendung von 0,310 g ($10^{-3}$ Mol) Triphenylphosphit anstelle von Triphenylphosphin. Nach einer Reaktionszeit von 8 Std. bei 130 °C erhält man 0,48 g (2,8 mMol) Zimtsäureäthylester, entsprechend einer Ausbeute von 11% d.Th. (Umsatzzahl 1100; Pd-Gehalt 0,01 Mol.%).

Beispiel 25:

Es wird wie in Beispiel 23 beschrieben vorgegangen unter Verwendung von 0,202 g ($10^{-3}$ Mol) Tri-n-butylphosphin anstelle von Triphenylphosphin. Nach einer Reaktionszeit von 8 Std. bei 130 °C erhält man 0,79 g (4,48 mMol) Zimtsäureäthylester, entsprechend einer Ausbeute von 18% d.Th. (Umsatzzahl 1800; Pd-Gehalt 0,01 Mol.%).

Beispiel 26:

Es wird wie in Beispiel 23 beschrieben vorgegangen, jedoch unter Verwendung von 0,304 g ($10^{-3}$ Mol) Tri-o-tolylphosphin anstelle von Triphenylphosphin. Nach einer Reaktionszeit von 8 Std. bei 130 °C erhält man 2,99 g (17,0 mMol) Zimtsäureäthylester, entsprechend einer Ausbeute von 68% d.Th. (Umsatzzahl 6800; Pd-Gehalt 0,01 Mol.%).

Beispiel 27:

Es wird wie in Beispiel 26 beschrieben vorgegangen, jedoch unter Verwendung von 5,0 g (25 mMol) 4-Brom-N,N-dimethylanilin und 2,99 ml (27,5 mMol) Acrylsäureäthylester. Nach einer Reaktionszeit von 23 Std. bei 130 °C und Aufarbeitung gemäss Beispiel 1 erhält man nach dem Umkristallisieren des Rohproduktes aus n-Hexan 0,60 g (2,8 mMol) 4-N,N-Dimethylaminozimtsäureäthylester als hellgelbe Kristalle vom Fp. 76 °C, entsprechend einer Ausbeute von 11% d.Th. (Umsatzzahl 1100; Pd-Gehalt 0,01 Mol.%).

Analyse für $C_{13}H_{17}NO_2$ (Molgewicht 219,28): berechnet C 71,21 H 7,82 N 6,39, gefunden C 71,47 H 7,85 N 6,47.

Beispiel 28:

Es wird wie in Beispiel 26 beschrieben vorgegangen, jedoch unter Verwendung von 4,28 g (25 mMol) 4-Bromtoluol und 2,99 ml (27,5 mMol) Acrylsäureäthylester. Nach einer Reaktionszeit von 23 Std. bei 130 °C erhält man 0,67 g (3,5 mMol) 4-Methylzimtsäureäthylester, entsprechend einer Ausbeute von 14% d.Th. (Umsatzzahl 1400; Pd-Gehalt 0,01 Mol.%).

Beispiel 29:

Es wird wie in Beispiel 26 beschrieben vorgegangen, jedoch unter Verwendung von 4,79 g (25 mMol) 4-Bromchlorbenzol und 2,99 ml (27,5 mMol) Acrylsäureäthylester. Nach einer Reaktionszeit von 8 Std. bei 130 °C erhält man 3,79 g (18,0 mMol) 4-Chlorzimtsäureäthylester, entsprechend einer Ausbeute von 72% d.Th. (Umsatzzahl 7200; Pd-Gehalt 0,01 Mol.%).

Beispiel 30:

Es wird wie in Beispiel 26 beschrieben vorgegangen, jedoch unter Verwendung von 5,05 g (25 mMol) 4-Bromnitrobenzol und 2,99 ml (27,5 mMol) Acrylsäureäthylester. Nach einer Reaktionszeit von 3 Std. bei 130 °C erhält man 4,86 g (22,0 mMol) 4-Nitrozimtsäureäthylester, entsprechend einer Ausbeute von 88% d.Th. (Umsatzzahl 8800; Pd-Gehalt 0,01 Mol.%).

Beispiel 31:

Es wird wie in Beispiel 26 beschrieben vorgegangen, jedoch unter Verwendung von 3,12 ml (25 mMol) 4-Bromanisol und 3,16 ml (27,5 mMol) Styrol. Nach einer Reaktionszeit von 23 Std. bei 130 °C auf Aufarbeitung gemäss Beispiel 1 erhält man nach dem Umkristallisieren des Rohproduktes aus n-Hexan 1,88 g (9,0 mMol) 4-Methoxystilben als weisse Kristalle vom Fp. 120 °C; Ausbeute 36% d.Th. (Umsatzzahl 3600; Pd-Gehalt 0,01 Mol.%).

Analyse für $C_{15}H_{14}O$ (Molgewicht 210,28): berechnet C 85,68 H 6,71, gefunden C 85,71 H 6,89.

Beispiel 32:

Es wird wie in Beispiel 26 beschrieben vorgegangen, jedoch unter Verwendung von 4,63 g (25 mMol) 4-Brombenzaldehyd und 3,16 ml (27,5 mMol) Styrol. Nach einer Reaktionszeit von 4 Std. bei 130 °C erhält man 3,74 g (18,0 mMol) 4-Formylstilben, entsprechend einer Ausbeute von 72% d.Th. (Umsatzzahl 7200; Pd-Gehalt 0,01 Mol.%).

Beispiel 33:

Es wird wie in Beispiel 26 beschrieben vorgegangen, jedoch unter Verwendung von 5,35 g (25 mMol) 4-Bromacetanilid und 2,99 ml (27,5 mMol) Acrylsäureäthylester. Nach einer Reaktionszeit von 12 Std. bei 130 °C und Aufarbeitung gemäss Beispiel 1 erhält man durch Umkristallisieren des Rohproduktes aus Toluol 0,60 g (2,6 mMol) 4-(N-Acetylamino)-zimtsäureäthylester als hellgelbe Kristalle vom Fp. 133 °C; Ausbeute 10% d.Th. (Umsatzzahl 1000; Pd-Gehalt

0,01 Mol.%). Analyse für $C_{13}H_{15}NO_3$ (Molgewicht 233): berechnet C 66,92 H 6,48 N 6,01 O 20,59, gefunden C 66,52 H 6,38 N 6,07 O 20,59.

Beispiel 34:

Es wird wie in Beispiel 26 beschrieben vorgegangen, jedoch unter Verwendung von 5,05 g (25 mMol) 4-Bromnitrobenzol und 3,41 ml (27,5 mMol) Crotonsäureäthylester. Nach einer Reaktionszeit von 23 Std. bei 130 °C erhält man nach Aufarbeitung gemäss Beispiel 1 und Umkristallisieren des Rohproduktes aus Methanol 2,59 g (11,0 mMol) 3-(4-Nitrophenyl)-corotonsäureäthylester als weisse Kristalle vom Fp. 75 °C; Ausbeute 44% d.Th. (Umsatzzahl 4400; Pd-Gehalt 0,01 Mol.%). Analyse für $C_{12}H_{13}NO_4$ (Molgewicht 235,24): berechnet C 61,27 H 5,57 N 5,96, gefunden C 61,19 H 5,66 N 6,11.

Beispiel 35:

Es wird wie in Beispiel 26 beschrieben vorgegangen, jedoch unter Verwendung von 5,05 g (25 mMol) 4-Bromnitrobenzol und 3,43 ml (27,5 mMol) Methacrylsäureäthylester. Nach einer Reaktionszeit von 8 Std. bei 130 °C und Aufarbeitung gemäss Beispiel 1 erhält man durch Umkristallisieren des Rohproduktes aus Methanol 2,70 g (11,5 mMol) α-Methyl-4-nitrozimtsäureäthylester als gelbe Kristalle vom Fp. 75 °C; Ausbeute 46% d.Th. (Umsatzzahl 4600; Pd-Gehalt 0,01 Mol.%). Analyse für $C_{12}H_{13}NO_4$ (Molgewicht 235,24): berechnet C 61,27 H 5,57 N 5,96, gefunden C 61,18 H 5,57 N 6,24.

Beispiel 36:

Zu 32 ml DMF werden unter Argon 0,0067 g ($8,0 \times 10^{-6}$ Mol) Diacetato-bis-(tris-o-tolylphosphin)palladium(II), 0,0087 g ($3,2 \times 10^{-5}$ Mol) Tri-o-tolylphosphin, 14,58 g (80 mMol) 3-Brombenzonitril, 5,86 ml (88 mMol) Acrylnitril und 7,22 g (88 mMol) wasserfreies Natriumacetat gegeben, und das Reaktionsgemisch wird 8 Std. bei 130 °C gerührt. Nach der Aufarbeitung gemäss Beispiel 1 und dem Umkristallisieren des Rohproduktes aus Isopropanol erhält man 10,72 g (69,6 mMol) 3-Cyanozimtsäurenitril als weisse Kristalle vom Fp. 139 °C; Ausbeute 87% d.Th. (Umsatzzahl 8700, Pd-Gehalt 0,01 Mol.%) Analyse für $C_{10}H_6N_2$ (Molgewicht 154,17): berechnet C 77,91 H 3,92 N 18,17, gefunden C 77,86 H 4,21 N 18,41.

Beispiel 37:

Es wird wie in Beispiel 26 beschrieben vorgegangen, jedoch unter Verwendung von 5,4 g (25 mMol) 2-Brom-4-nitrotoluol und 1,83 ml (27,5 mMol) Acrylnitril. Nach einer Reaktionszeit von 8 Std. bei 130 °C und Aufarbeitung gemäss Beispiel 1 erhält man nach dem Umkristallisieren des Rohproduktes aus Toluol/Isopropanol 2,53 g (13,45 mMol) 2-Methyl-4-nitrozimtsäurenitril als weisse nadelförmige Kristalle vom Fp 166 °C; Ausbeute 54% d.Th. (Umsatzzahl 5400; Pd-Gehalt 0,02 Mol.%). Analyse für $C_{10}H_8N_2O_2$ (Molgewicht 188,19): berechnet C 63,83 H 4,29 N 14,89 O 17,00, gefunden C 64,17 H 4,48 N 15,00 O 17,11.

Beispiel 38:

Zu 18 ml DMF werden 2 ml Stammlösung gemäss Beispiel 23 gegeben. Danach werden 9,26 g (50 mMol) 4-Brombenzaldehyd, 5,58 ml (55 mMol) Cyclohexen und 4,52 g (55 mMol) wasserfreies Natriumacetat zugefügt, und das Reaktionsgemisch wird 8 Std. bei 140 °C in einem Druckrohr gerührt. Nach Aufarbeitung gemäss Beispiel 1 wird das Rohprodukt im Vakuum destilliert. Man erhält 2,05 g (11,0 mMol) 4-Formylphenylcyclohexen als gelbe Flüssigkeit vom Sdp. 140–150 °C/2400 Pa; Ausbeute 22% d.Th. (Umsatzzahl 2200; Pd-Gehalt 0,01 Mol.%). Das Produkt ist ein Isomerengemisch.

Beispiel 39:

Es wird wie in Beispiel 26 beschrieben vorgegangen, jedoch unter Verwendung von 5,05 g (25 mMol) 4-Bromnitrobenzol und 4,33 ml (27,5 mMol) 1-Octen. Nach einer Reaktionszeit von 10 Std. bei 130 °C und Aufarbeitung gemäss Beispiel 1 erhält man durch Destillation des Rohproduktes 3,90 g (16,7 mMol) 4-Nitrophenylocten vom Sdp. 140–150 °C/27 Pa als gelbe Flüssigkeit; Ausbeute 67% d.Th. (Umsatzzahl 6700; Pd-Gehalt 0,01 Mol.%). Das Produkt ist ein Isomerengemisch. Analyse für $C_{14}H_{19}NO_2$ (Molgewicht 233,21): berechnet C 70,08 H 8,21 N 6,01 gefunden C 71,75 H 8,10 N 6,16.

Beispiel 40:

Zu 18 ml DMF werden 2 ml der Stammlösung gemäss Beispiel 23 gegeben. Danach werden 9,77 g (50 mMol) 4-Chlorbrombenzol und 4,52 g (55 mMol) wasserfreies Natriumacetat zugefügt. Das Reaktionsgemisch wird dann in einer Druckapparatur unter Äthylen gesetzt und bei 10 bar und 130 °C 6 Std. gerührt. Man erhält 3,62 g (26,1 mMol) 4-Chlorstyrol entsprechend einer Ausbeute von 52% d.Th. (Umsatzzahl 5200; Pd-Gehalt 0,1 Mol.%).

Beispiel 41:

Es wird wie in Beispiel 9 beschrieben vorgegangen, jedoch unter Verwendung von 4,63 g (25 mMol) 4-Brombenzaldehyd, 2,26 g (27,5 mMol) wasserfreiem Natriumacetat und Äthylen. Das Reaktionsgemisch wird während 4 Std. bei 130 °C gerührt, unter Durchleitung des Äthylens bei Normaldruck. Nach der Aufarbeitung gemäss Beispiel 1 und dem Umkristallisieren des Rohprodukts aus Isopropanol erhält man 1,73 g (7,33 mMol) 4,4'-Diformylstilben als gelbe Kristalle vom Fp. 172 °C; Ausbeute 59% d.Th. (Umsatzzahl 5900, bezogen auf den 4-Brombenzaldehyd; Pd-Gehalt 0,01 Mol.%). Analyse für $C_{16}H_{12}O_2$ (Molgewicht 236,27: berechnet C 81,34 H 5,12 O 13,54, gefunden C 81,20 H 5,36 O 13,46.

Beispiel 42

Zu 90 ml DMF werden unter Argon 0,00505 g ($2,25 \times 10^{-5}$ Mol) Palladiumacetat, 0,0274 g ($9 \times 10^{-5}$ Mol) Tri-o-tolylphosphin, 46,8 g (0,225 Mol) 4-Brom-zimtsäurenitril und 20,3 g (0,2475 Mol) wasserfreies Natriumacetat gegeben. Danach wird Äthylen bei Normaldruck eingeleitet, und das Reaktionsgemisch wird 10 Std. bei 130 °C gerührt. Das Rohprodukt wird im Soxhlet mit Toluol extrahiert und anschliessend aus DMF/Wasser umkristallisiert. Man erhält 21,3 g (0,0755 Mol) des optischen Aufhellers 4,4'-Bis-(trans-2-cyanovinyl)-trans-Stilben als gelbe Kristalle vom Fp. 219–221 °C; Ausbeute 67% d.Th. (Umsatzzahl 6700 bezogen auf den Bromaromaten; Pd-Gehalt 0,01 Mol.%). Analyse für $C_{20}H_{14}N_2$ (Molgewicht 282): berechnet C 85,08 H 5,00 N 9,92, gefunden C 84,75 H 5,07 N 9,67.

Beispiel 43:
Es wird wie in Beispiel 26 beschrieben vorgegangen, jedoch unter Verwendung von 3,49 ml (25 mMol) 1-Bromnaphthalin, 3,16 g (27,5 mMol) Styrol und 2,70 g (27,5 mMol) Kaliumacetat. Nach einer Reaktionszeit von 17 Std. bei 130 °C und Aufarbeitung gemäss Beispiel 1 erhält man nach dem Umkristallisieren des Rohproduktes aus Äthanol 2,3 g (10,0 mMol) 1-(2-Phenylvinyl)-naphthalin als grünlich-gelbe Kristalle vom Fp. 71 °C; Ausbeute 40% (Umsatzzahl 4000; Pd-Gehalt 0,01 Mol). Analyse für $C_{18}H_{14}$ (Molgewicht 230,31): berechnet C 93,88 H 6,13, gefunden C 93,65 H 6,11.

Beispiel 44:
Es wird wie in Beispiel 9 beschrieben vorgegangen, jedoch unter Verwendung von 2,79 ml (25 mMol) Jodbenzol und 2,98 ml (27,5 mMol) Acrylsäureäthylester. Nach einer Reaktionszeit von 4,5 Std. bei 130 °C erhält man 2,92 g (16,6 mMol) Zimtsäureäthylester, entsprechend einer Ausbeute von 66% d.Th. (Umsatzzahl 6600; Pd-Gehalt 0,01 Mol.%).

Beispiel 45:
Es wird wie in Beispiel 46 beschrieben vorgegangen, jedoch ohne Verwendung von Phosphin. Nach einer Reaktionszeit von 4,5 Std. bei 130 °C erhält man 2,71 g (15,4 mMol) Zimtsäureäthylester, entsprechend einer Ausbeute von 62% d.Th. (Umsatzzahl 6200; Pd-Gehalt 0,01 Mol.%).

Beispiel 46:
Es wird wie in Beispiel 47 beschrieben vorgegangen, jedoch unter Verwendung von 7,07 g (25 mMol) 4-Bromjodbenzol und 1,83 g (27,5 mMol) Acrylnitril. Nach einer Reaktionszeit von 8 Std. bei 130 °C erhält man 3,95 g (19,0 mMol) 4-Bromzimtsäurenitril, entsprechend einer Ausbeute von 76% d.Th. (Umsatzzahl 7600; Pd-Gehalt 0,01 Mol.%).

Beispiel 47:
Unter Argon wird eine Stammlösung aus 0,1041 g ($1,25 \times 10^{-4}$ Mol) Diacetato-bis-(tris-o-tolylphosphin)-palladium (II) in 50 ml Hexamethylphosphorsäuretriamid hergestellt. Zu 19 ml Hexamethylphosphorsäuretriamid werden unter Argon 1 ml Stammlösung, 9,25 g (50 mMol) 4-Brombenzaldehyd, 3,66 ml (55 mMol) Acrylnitril, und 4,51 g (55 mMol) wasserfreies Natriumacetat gegeben und das Gemisch wird 8 Stunden bei 130 °C gerührt. Das Hexamethylphosphorsäuretriamid wird unter Hochvakuum entfernt und das Produkt destilliert. Man erhält 6,58 g (41,9 mMol) 4-Formyl-zimtsäurenitril, entsprechend einer Ausbeute von 84% d.Th. (Umsatzzahl 16 800; Pd-Gehalt 0,005 Mol%).

Beispiel 48:
Unter Argon wird eine Stammlösung aus 0,02244 g ($10^{-4}$ Mol) Palladiumacetat und 0,1216 g ($4 \times 10^{-4}$ Mol) Tri-o-tolylphosphin in 20 ml Hexamethylphosphorsäuretriamid hergestellt. Zu 9,5 ml Hexamethylphosphorsäuretriamid werden unter Äthylen 0,5 ml Stammlösung, 5,2 g (25 mMol) 4-Brom-zimtsäurenitril und 2,26 g (27,5 mMol) wasserfreies Natriumacetat gegeben und das Gemisch wird 8 Stunden bei 130 °C gerührt, während Äthylen ständig über die Oberfläche geleitet wird. Anschliessend wird das Gemisch mit 50 ml Wasser versetzt, das Produkt abfiltriert und mit 50 ml Methanol gewaschen. Nach Umkristallisieren aus 120 ml Propionitril erhält man 2,02 g (7,2 mMol) 4,4'-Bis-(2-cyanovinyl)-stilben, entsprechend einer Ausbeute von 57% d.Th. (Umsatzzahl 5700; Pd-Gehalt 0,01 Mol%) bezogen auf Bromaromat.

Beispiel 49:
Unter Argon wird eine Stammlösung von 0,01122 g ($5 \times 10^{-5}$ Mol) Palladiumacetat und 0,0608 g ($2 \times 10^{-4}$ Mol) Tri-o-tolylphosphin in 20 ml N,N-Dimethylformamid (DMF) hergestellt. Zu 9 ml DMF werden unter Argon 1 ml Stammlösung, 2,90 ml (25 mMol) 2-Brombenzaldehyd, 2,98 ml (27,5 mMol) Acrylsäureäthylester und 2,26 g (27,5 mMol) wasserfreies Natriumacetat gegeben und das Reaktionsgemisch wird 6 Stunden bei 130 °C gerührt. Nach der Aufarbeitung gemäss Beispiel 1, wird das Rohprodukt im Hochvakuum destilliert. Man erhält 2,1 g (10,3 mMol) o-Formylzimtsäureäthylester als hellbraune Flüssigkeit vom S.p. 126–128 °C/0,4 mm entsprechend einer Ausbeute von 41% d.Th. (Umsatzzahl 4100; Pd-Gehalt 0,01 Mol%).

Beispiel 50:
Unter Argon werden zu 16,3 ml DMF, 0,67 ml der Stammlösung von Beispiel 52, 3,05 g (16,8 mMol) 2-Brom-benzonitril, 1,99 ml (18,4 mMol) Acrylsäureäthylester und 1,50 g (18,4 mMol) wasserfreies Natriumacetat gegeben und das Reaktionsgemisch 3 Stunden bei 130 °C gerührt. Nach der Aufarbeitung gemäss Beispiel 1 wird das Rohprodukt aus einem Pentan/Tetrachlorkohlenstoff-Gemisch umkristallisiert. Man erhält 1,5 g (7,5 mMol) o-Cyanozimtsäureäthylester als weisse Kristalle vom Fp. 56 °C entsprechend einer Ausbeute von 44% d.Th. (Umsatzzahl 4400; Pd-Gehalt 0,01 Mol%). Analyse für $C_{12}H_{11}NO_2$ (Molgewicht 201,23): berechnet C 71,63, H 5,51; N 6,96; O 15,90 – gefunden C 71,17; H 5,64; N 6,88; O 16,56.

Beispiel 51:
Unter Argon wird eine Stammlösung von 0,0168 g ($7,5 \times 10^{-5}$ Mol) Palladiumacetat und

0,0912 g ($3 \times 10^{-4}$ Mol) Tri-o-tolyl-phosphin in 50 ml DMF hergestellt. Zu 14 ml DMF werden unter Argon 1 ml Stammlösung, 4,71 g (15 mMol) o-Brombenzolsulfonsäurephenylester, 1,79 ml (16,5 mMol) Acrylsäureäthylester, und 1,35 g (16,5 mMol) wasserfreies Natriumacetat zugegeben und das Reaktionsgemisch wird 5 Stunden bei 130 °C gerührt. Nach der Aufarbeitung gemäss Beispiel 1 wird das Rohprodukt im Hochvakuum destilliert. Man erhält 2,54 g (7,65 mMol) o-Phenoxysulfonylzimtsäureäthylester als farblose Flüssigkeit vom Sp. 186–190 °C/0,35 mm entsprechend einer Ausbeute von 51% d.Th. (Umsatzzahl 5100; Pd-Gehalt 0,01 Mol%).

Analyse für $C_{17}H_{16}SO_5$ (Molgewicht 332,37): berechnet C 61,44; H 4,85; O 24,07; S 9,65 – gefunden C 61,37; H 4,84; O 24,22; S 9,65.

Verwendungsbeispiel 52:

Zu einer Lösung von 17,5 g 2-(4-Diäthoxyphosphorylmethylphenyl)-4-methoxy-6-methyl-pyrimidin und 7,8 g 4-Formylzimtsäurenitril in 100 ml DMF tropft man bei Raumtemperatur innerhalb von 16 Minuten 11 g einer 30%igen Natriummethylat-Lösung zu. Die Temperatur steigt bis auf 40 °C. Das Reaktionsgemisch wird hierauf 2 Stunden bei 45 °C verrührt, auf ein Gemisch, bestehend aus 160 ml Methanol und 250 ml Wasser, gegossen und die wässrige Suspension wird mit Essigsäure auf pH 7 gestellt. Der Niederschlag wird abgenutscht, mit Wasser gewaschen und getrocknet. Nach mehrmaliger Umkristallisation aus Toluol/Ligroin (1:1) werden unter Zuhilfenahme von Bleicherde 12,1 g, entsprechend 62% der Theorie, des optischen Aufhellers der Formel

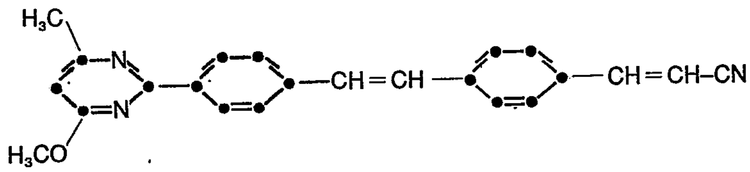

als gelbliches Pulver vom Smp. 196–197 °C erhalten.

Vergleichsbeispiele

Nach dem aus der US-B-3 922 299 bekannt gewordenen Verfahren werden die Versuche a) bis c) durchgeführt, und zwar indem man zu 10 ml Propionitril unter Argon in einem Druckrohr 4,63 g (25 mMol) 4-Brombenzaldehyd, 2,08 ml (31,25 mMol) Acrylnitril, 4,36 ml (31,25 mMol) Triäthylamin und die unten angegebenen Mengen Palladium(II)acetat und Tri-o-tolylphosphin gibt. Das Rohr wird geschlossen und der Inhalt bei 140 °C gerührt. Man erhält als Produkt 4-Formylzimtsäurenitril. Die Reaktionszeiten und Ausbeuten sind in der folgenden Tabelle angegeben.

| $Pd(OAc)_2$ (mMol) | $P(o\text{-}Tolyl)_3$ (mMol) | Zeit (Std.) | Ausbeute (%) | Umsatzzahl |
|---|---|---|---|---|
| a) 0,25 | 1,00 | 6 | 92 | 92 |
| b) $2,5 \times 10^{-2}$ | $10^{-1}$ | 23 | 47 | 470 |
| c) $2,5 \times 10^{-3}$ | $10^{-2}$ | 23 | < 2 | <200 |
| Zum Vergleich die Daten aus Beispiel 21 | | | | |
| d) $2,5 \times 10^{-4}$ | $5 \times 10^{-4}$ | 24 | 79 | 79.000 |

**Patentansprüche**

1. Verfahren zur Herstellung von Verbindungen der Formel I

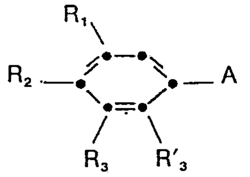 (I),

worin A $C_{2-12}$-Alkenyl, $C_{4-8}$-Cycloalkenyl oder eine Gruppe 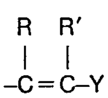 darstellt,

Y –CN, –COR″, –COOH, –COOR″, –CON(R″)$_2$ oder

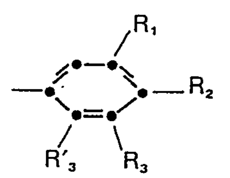 ,

R Wasserstoff, Methyl, –CN oder –COOR″, R′ Wasserstoff, Methyl oder –$CH_2COOR''$, R″ $C_{1-12}$-Alkyl oder Phenyl und $R_1$ und $R_3$ unabhängig voneinander Wasserstoff, Phenyl, $C_{1-8}$-Alkyl, $C_{1-5}$-Alkoxy, –$CH(OCH_3)_2$, –$CH(OC_2H_5)_2$,

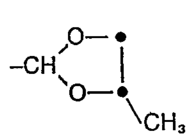 , 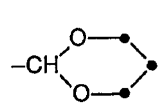 , 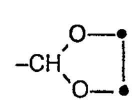 ,

$-OH$, Cl, F, $-NO_2$, $-CN$, $-CHO$, $-CO-C_{1-4}$-Alkyl, $-COO-C_{1-4}$-Alkyl, $-NHCO-C_{1-4}$-Alkyl, $-NH_2$, $-NH-C_{1-4}$-Alkyl, $-N(C_{1-4}\text{-Alkyl})_2$ oder $-SO_3^-M^+$ bedeuten, $R_2$ dieselbe Bedeutung wie $R_1$ bzw. $R_3$ haben kann oder eine Gruppe $-CH=N$-Phenyl oder $-C(R_4)=C(R_5)(R_6)$ darstellt und $R'_3$ Wasserstoff, Methyl, $-CN$, $-CHO$ oder $-SO_3C_6H_5$ ist oder $R_1$ und $R_2$ Wasserstoff bedeuten und $R_3$ und $R'_3$ zusammen $-CH=CH-CH=CH-$ darstellen, $R_4$ Wasserstoff, $C_{1-4}$-Alkyl, $-CN$ oder $-COO-C_{1-4}$-Alkyl, $R_5$ Wasserstoff, $C_{1-4}$-Alkyl, $-NHCHO$, $-(CH_2)_m-COO-C_{1-4}$-Alkyl oder $-(CH_2)_m-CN$ mit $m = 1$ bis 4, $R_6$ $-CN$ oder $-COO-C_{1-4}$-Alkyl und $M^+$ ein Metallkation bedeuten, dadurch gekennzeichnet, dass man eine Verbindung der Formel II

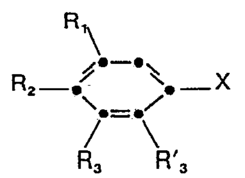

(II)

in Gegenwart eines Alkalimetall- oder Erdalkalimetallsalzes einer aliphatischen Monocarbonsäure mit 1–12 C-Atomen oder der Benzoesäure, eines cyclischen oder N,N-disubstituierten Amids als Lösungsmittel und einer gegebenenfalls arsen- oder phosphorhaltigen Palladium-Verbindung als Katalysator mit einer Verbindung der Formel III

HA (III)

umsetzt, wobei für A, Y, R, R', $R_1$, $R_2$, $R_3$ und $R'_3$ das unter Formel I Angegebene gilt, X Jod oder Brom bedeutet und der Palladiumgehalt 0,1 bis 0,0001 Mol.%, bezogen auf die Verbindung der Formel II, beträgt.

2. Verfahren zur Herstellung von Verbindungen der Formel (I)

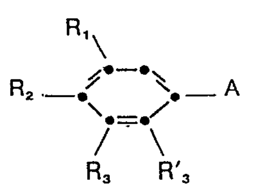

worin A die Gruppe

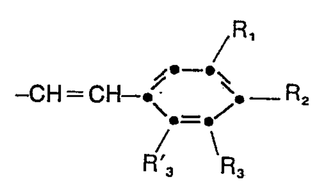

darstellt und $R_1$ und $R_3$ unabhängig voneinander Wasserstoff, Phenyl, $C_{1-8}$-Alkyl, $C_{1-5}$-Alkoxy, $-CH(OCH_3)_2$, $-CH(OC_2H_5)_2$,

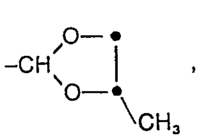 , 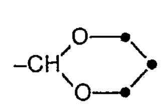 , 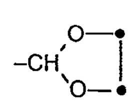 ,

$-OH$, Cl, F, $-NO_2$, $-CN$, $-CHO$, $-CO-C_{1-4}$-Alkyl, $-COO-C_{1-4}$-Alkyl, $-NHCO-C_{1-4}$-Alkyl, $-NH_2$, $-NH-C_{1-4}$-Alkyl, $-N(C_{1-4}\text{-Alkyl})_2$ oder $-SO_3^-M^+$ bedeuten, $R_2$ dieselbe Bedeutung wie $R_1$ bzw. $R_3$ haben kann oder eine Gruppe $-CH=N$—Phenyl oder $-C(R_4)=C(R_5)(R_6)$ darstellt und

$R'_3$ Wasserstoff, Methyl, $-CN$, $-CHO$ oder $-SO_3C_6H_5$ ist oder $R_1$ und $R_2$ Wasserstoff bedeuten und $R_3$ und $R'_3$ zusammen $-CH=CH-CH=CH-$ darstellen,

$R_4$ Wasserstoff, $C_{1-4}$-Alkyl, $-CN$ oder $-COO-C_{1-4}$-Alkyl,

$R_5$ Wasserstoff, $C_{1-4}$-Alkyl, $-NHCHO$, $-(CH_2)_m-COO-C_{1-4}$-Alkyl oder $-(CH_2)_m-CN$ mit $m = 1$ bis 4,

$R_6$ $-CN$ oder $-COO-C_{1-4}$-Alkyl und

$M^+$ ein Metallkation bedeuten, dadurch gekennzeichnet, dass man eine Verbindung der Formel II

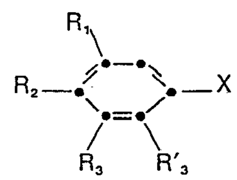

(II)

worin X Jod oder Brom bedeutet und $R_1$, $R_2$, $R_3$ und $R'_3$ die oben angegebene Bedeutung haben in Gegenwart eines Alkalimetall- oder Erdalkalimetallsalzes einer aliphatischen Monocarbonsäure mit 1–12 C-Atomen oder der Benzoesäure, eines cyclischen oder N,N-disubstituierten Amids als Lösungsmittel und einer gegebenenfalls arsen- oder phosphorhaltigen Palladium-Verbindung als Katalysator und bei einem Druck zwischen 0,1 und 1 bar mit Äthylen umsetzt, wobei der Palladiumgehalt 0,1 bis 0,0001 Mol.%, bezogen auf die Verbindung der Formel II, beträgt.

3. Verfahren nach Patentansprüchen 1 und 2, dadurch gekennzeichnet, dass der Palladiumgehalt 0,05 bis 0,0001 Mol.%, insbesondere 0,01 bis 0,001 Mol.%, bezogen auf die Verbindung der Formel II, beträgt.

4. Verfahren nach Patentanspruch 1, dadurch gekennzeichnet, dass man eine Verbindung der Formel III verwendet, worin A $-C(R)=C(R')-Y$ darstellt und mindestens eines von R und R' Wasserstoff ist.

5. Verfahren nach Patentanspruch 1, dadurch gekennzeichnet, dass man eine Verbindung der Formel II verwendet, worin X Brom, $R'_3$ Wasserstoff, $R_1$ und $R_3$ unabhängig voneinander Wasserstoff, Phenyl, $C_{1-3}$-Alkyl, besonders Methyl, Methoxy, $-NO_2$, $-CN$, $-CHO$, Cl, $-NHCO-C_{1-2}$-Alkyl, $-COO-C_{1-2}$-Alkyl, $-CO-C_{1-2}$-Alkyl oder $-N(C_{1-2}\text{-Alkyl})_2$ bedeuten und $R_2$ dieselbe Bedeutung wie $R_1$ bzw. $R_3$ haben kann oder eine Gruppe $-C(R_4)=C(R_5)(R_6)$ darstellt, worin $R_4$ Wasserstoff, $-COOCH_3$ oder $-COOC_2H_5$, $R_5$ Wasserstoff, Methyl, Äthyl, $-CH_2COOCH_3$, $-CH_2COOC_2H_5$ oder $-CH_2CH_2CN$ und $R_6$ $-CN$, $-COOCH_3$ oder $-COOC_2H_5$ bedeuten, wobei eines von $R_4$ und $R_5$ Wasserstoff ist.

6. Verfahren nach Patentanspruch 1, dadurch gekennzeichnet, dass man eine Verbindung der

Formel II verwendet, worin X Brom, $R_1$, $R_3$ und $R'_3$ Wasserstoff und $R_2$ Wasserstoff, Phenyl, Methyl, Methoxy, $-CN$, $-NO_2$, $-CHO$, Cl, $NHCOCH_3$, $-N(CH_3)_2$ oder eine Gruppe $-CH=CH-R_6$ und $R_6$ $-CN$, $-COOCH_3$ oder $-COOC_2H_5$ darstellen.

7. Verfahren nach Patentanspruch 1, dadurch gekennzeichnet, dass man eine Verbindung der Formel III verwendet, worin A Alkylen mit 2–8 C-Atomen, Cyclopentylen, Cyclohexylen oder eine Gruppe $-C(R)=C(R')-Y$ darstellt, eines von R und R' Wasserstoff und das andere Methyl oder beide Wasserstoff und Y Phenyl, $-CN$ oder $-COOR''$ mit $R''=$ Phenyl oder $C_{1-4}$-Alkyl, besonders Methyl oder Äthyl, bedeuten.

8. Verfahren nach Patentanspruch 1, dadurch gekennzeichnet, dass man als Verbindung der Formel III Äthylen, Acrylnitril, Acrylsäuremethyl- oder -äthylester verwendet.

9. Verfahren nach Patentanspruch 2, dadurch gekennzeichnet, dass man eine Verbindung der Formel

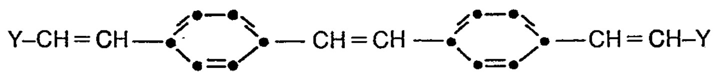

herstellt, worin Y $-COOC_2H_5$ und insbesondere $-CN$ darstellt.

10. Verfahren nach Patentanspruch 1, dadurch gekennzeichnet, dass man 4-Formylzimtsäurenitril herstellt.

11. Verfahren nach Patentanspruch 2, dadurch gekennzeichnet, dass man 4,4'-Stilbendialdehyd herstellt.

12. Verfahren nach Patentansprüchen 1 und 2, dadurch gekennzeichnet, dass man als Base ein Alkalimetall- oder Erdalkalimetallacetat, -propionat oder -benzoat verwendet.

13. Verfahren nach Patentansprüchen 1 und 2, dadurch gekennzeichnet, dass man als Base Kaliumacetat und insbesondere Natriumacetat verwendet.

14. Verfahren nach Patentansprüchen 1 und 2, dadurch gekennzeichnet, dass man als Amid eine Verbindung der Formel IV

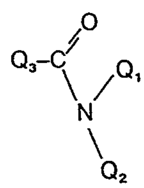 (IV)

verwendet, worin $Q_1$ und $Q_2$ unabhängig voneinander geradkettiges oder verzweigtes $C_{1-8}$-Alkyl, $C_{5-8}$-Cycloalkyl oder Benzyl oder zusammen $-(CH_2)_3-$, $-(CH_2)_4-$ oder $-(CH_2)_2-O-(CH_2)_2-$ darstellen und $Q_3$ Wasserstoff, geradkettiges oder verzweigtes $C_{1-8}$-Alkyl oder zusammen mit $Q_1$ $-(CH_2)_q-$ mit $q=3$, 4 oder 5 bedeutet.

15. Verfahren nach Patentansprüchen 1 und 2, dadurch gekennzeichnet, dass man als Lösungsmittel N,N-Dimethylformamid, N,N-Diäthylformamid, N,N-Dimethylacetamid, N-Methylpyrrolidon oder Hexamethylphosphorsäuretriamid verwendet.

16. Verfahren nach Patentansprüchen 1 und 2, dadurch gekennzeichnet, dass man als Katalysator ein Gemisch aus $PdCl_2$, Palladiumacetat oder Bis-(dibenzylidenaceton)palladium(O) und Tri-n-butylphosphin, Triphenylphosphin, Tri-o-tolylphosphin oder Triphenylphosphit verwendet.

17. Verfahren nach Patentansprüchen 1 und 2, dadurch gekennzeichnet, dass man als Katalysator ein Gemisch aus Palladiumacetat und Triphenylphosphin oder Tri-o-tolylphosphin und insbesondere Diacetato-bis-(tris-o-tolylphosphin)palladium(II) verwendet.

18. Verfahren nach Patentansprüchen 1 und 2, dadurch gekennzeichnet, dass man in N,N-Dimethylformamid als Lösungsmittel arbeitet, Kalium- oder Natriumacetat als Base und als Katalysator ein Gemisch aus Palladiumacetat und Triphenylphosphin oder Tri-o-tolylphosphin und insbesondere Diaceto-bis-(tris-o-tolylphosphin)palladium(II) verwendet.

**Claims**

1. A process for the preparation of a compound of the formula I

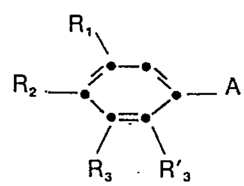 (I),

in which A is $C_{2-12}$-alkenyl, $C_{4-8}$-cycloalkenyl or a

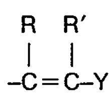

group, Y is $-CN$, $-COR''$, $-COOH$, $-COOR''$, $-CON(R'')_2$ or

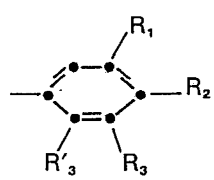 ,

R is hydrogen, methyl, $-CN$ or $-COOR''$, R' is hydrogen, methyl or $-CH_2COOR''$, R'' is $C_{1-12}$-alkyl or phenyl and each of $R_1$ and $R_3$ independently of the other is hydrogen, phenyl, $C_{1-8}$-alkyl, $C_{1-5}$-alkoxy, $-CH(OCH_3)_2$, $-CH(OC_2H_5)_2$,

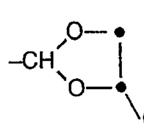 , 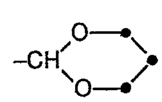 , 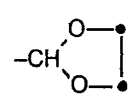 ,

–OH, Cl, F, $-NO_2$, –CN, –CHO, $-CO-C_{1-4}$-alkyl, $-COO-C_{1-4}$-alkyl, $-NHCO-C_{1-4}$-alkyl, $-NH_2$, $-NH-C_{1-4}$-alkyl, $-N(C_{1-4}\text{-alkyl})_2$ or $-SO_3^-M^+$, $R_2$ can have the same meaning as $R_1$ or $R_3$ or is a –CH = N-phenyl or $-C(R_4) = C(R_5)(R_6)$ group and $R'_3$ is hydrogen, methyl, –CN, –CHO or $-SO_3$-phenyl or $R_1$ and $R_2$ are hydrogen and $R_3$ and $R'_3$ together are –CH = CH–CH = CH–, $R_4$ is hydrogen, $C_{1-4}$-alkyl, –CN or $-COO-C_{1-4}$-alkyl, $R_5$ is hydrogen, $C_{1-4}$-alkyl, –NHCHO, $-(CH_2)_m-COO-C_{1-4}$-alkyl or $-(CH_2)_m-CN$ in which m = 1 to 4, $R_6$ is –CN or $-COO-C_{1-4}$-alkyl and $M^+$ is a metal cation, which process comprises reacting a compound of the formula II

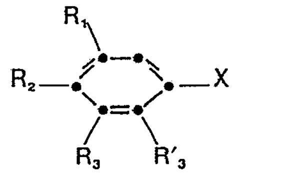

(II)

with a compound of the formula III

HA (III)

in the presence of an alkali metal salt or alkaline earth metal salt of an aliphatic monocarboxylic acid having 1–12 C atoms or of benzoic acid, a cyclic or N,N-disubstituted amide, as the solvent, and of a palladium compound which may contain arsenic or phosphorus, as the catalyst, A, Y, R, R′, $R_1$, $R_2$, $R_3$ and $R'_3$ being as defined under formula I, X being iodine or bromine and the palladium content being 0.1 to 0.0001 mol%, based on the compound of the formula II.

2. A process for the preparation of a compound of the formula I

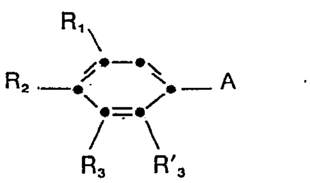

in which A is the

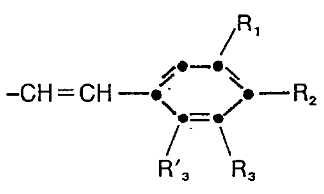

group, and each of $R_1$ and $R_3$ independently of the other is hydrogen, phenyl, $C_{1-8}$-alkyl, $C_{1-5}$-alkoxy, $-CH(OCH_3)_2$, $-CH(OC_2H_5)_2$

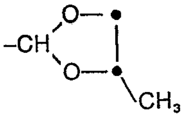 , 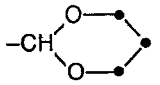 , 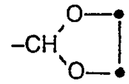 ,

–OH, Cl, F, $-NO_2$, –CN, –CHO, $-CO-C_{1-4}$-alkyl, $-COO-C_{1-4}$-alkyl, $-NHCO-C_{1-4}$-alkyl, $-NH_2$, $-NH-C_{1-4}$-alkyl, $-N(C_{1-4}\text{-alkyl})_2$ or $-SO_3^-M^+$, $R_2$ can have the same meaning as $R_1$ or $R_3$ or is a –CH = N-phenyl or $-C(R_4) = C(R_5)(R_6)$ group and $R'_3$ is hydrogen, methyl, –CN, –CHO or $-SO_3$-phenyl or $R_1$ and $R_2$ are hydrogen and $R_3$ and $R'_3$ together are –CH = CH–CH = CH–, $R_4$ is hydrogen, $C_{1-4}$-alkyl, –CN or $-COO-C_{1-4}$-alkyl, $R_5$ is hydrogen, $C_{1-4}$-alkyl, –NHCHO, $-(CH_2)_m-COO-C_{1-4}$-alkyl or $-(CH_2)_m-CN$ in which m = 1 to 4, $R_6$ is –CN or $-COO-C_{1-4}$-alkyl and $M^+$ is a metal cation, which process comprises reacting a compound of the formula II

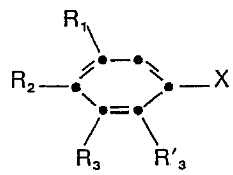

(II)

in which X is iodine or bromine and $R_1$, $R_2$, $R_3$ and $R'_4$ are as defined above, with ethylene, in the presence of an alkali metal salt or alkaline earth metal salt of an aliphatic monocarboxylic acid having 1–12 C atoms or of benzoic adic, a cyclic or N,N-disubstituted amide, as the solvent, and of a palladium compound which may contain arsenic or phosphorus, as the castlyst, and under a pressure in the range from 0.1 to 1 bar, the palladium content being 0.1 to 0.0001 mol%, based on the compound of the formula II.

3. A process according to either of claims 1 or 2, wherein the palladium content is 0.05 to 0.0001 mol%, in particular 0.01 to 0.001 mol%, based on the compound of the formula II.

4. A process according to claim 1, which comprises the use of a compound of the formula III in which A is –C(R) = C(R′)–Y and at least one of R and R′ is hydrogen.

5. A process according to claim 1, which comprises the use of a compound of the formula II in which X is bromine, $R'_3$ is hydrogen, each of $R_1$ and $R_3$ independently of the other is hydrogen, phenyl, $C_{1-3}$-alkyl, particularly methyl, methoxy, $-NO_2$, –CN, –CHO, Cl, $-NHCO-C_{1-2}$-alkyl, $-COO-C_{1-2}$-alkyl, $-CO-C_{1-2}$-alkyl or $-N(C_{1-2}\text{-alkyl})_2$, and $R_2$ can have the same meaning as $R_1$ or $R_3$ or is a $-C(R_4) = C(R_5)(R_6)$ group in which $R_4$ is hydrogen, $-COOCH_3$, or $-COOC_2H_5$, $R_5$ is hydrogen, methyl, ethyl, $-CH_2COOCH_3$, $-CH_2COOC_2H_5$ or $-CH_2CH_2CN$ and $R_6$ is –CN, $-COOCH_3$ or $-COOC_2H_5$, one of $R_4$ and $R_5$ being hydrogen.

6. A process according to claim 1, which comprises the use of a compound of the formula II in which X is bromine, $R_1$, $R_3$ and $R'_3$ are hydrogen and $R_2$ is hydrogen, phenyl, methyl, methoxy, –CN, $-NO_2$, –CHO, Cl, $-NHCOCH_3$, $-N(CH_3)_2$ or a $-CH = CH-R_6$ group and $R_6$ is –CN, $-COOCH_3$ or $-COOC_2H_5$.

7. A process according to claim 1, which comprises the use of a compound of the formula III in which A is alkylene having 2–8 C atoms, cyclopentylene, cyclohexylene or a –C(R) = C(R′)–Y group, one of R and R′ is hydrogen and the other is methyl or both are hydrogen, and Y is phenyl, –CN or –COOR″ in which R″ = phenyl or $C_{1-4}$-alkyl, particularly methyl or ethyl.

8. A process according to claim 1, wherein the compound of the formula III is ethylene, acrylonitrile, methyl acrylate or ethyl acrylate.

9. A process according to claim 2, wherein a compound ot the formula

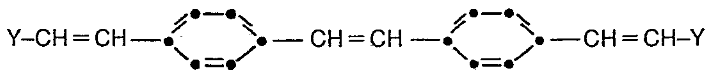 ,

wherein Y is $-COOC_2H_5$ and especially –CN, is prepared.

10. A process according to claim 1, wherein 4-formylcinnamonitrile is prepared.

11. A process according to claim 2, wherein 4,4'-stilbenedialdehyde is prepared.

12. A process according to either of claims 1 or 2, wherein the base is an alkali metal acetate, propionate or benzoate or an alkaline earth metal acetate, propionate or benzoate.

13. A process according to either of claims 1 or 2, wherein the base is potassium acetate and, in particular, sodium acetate.

14. A process according to either of claims 1 or 2, wherein the amide is a compound of the formula IV

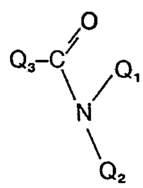 (IV)

in which each of $Q_1$ and $Q_2$ independently of the other is straight chain or branched $C_{1-8}$-alkyl, $C_{5-8}$cycloalkyl or benzyl or together are $-(CH_2)_3-$, $-(CH_2)_4-$ or $-(CH_2)_2-O-(CH_2)_2-$ and $Q_3$ is hydrogen or straight chain or branched $C_{1-8}$-alkyl or, together with $Q_1$, is $-(CH_2)_q-$ in which q is 3, 4 or 5.

15. A process according to either of claims 1 or 2, wherein the solvent is N,N-dimethylformamide, N,N-diethylformamide, N,N-dimethylacetamide, N-methylpyrrolidone or hexamethylphosphoric acid triamide.

16. A process according to either of claims 1 or 2, wherein the catalyst is a mixture consisting of $PdCl_2$, palladium acetate or bis(dibenzylideneacetone)palladium(O) and tri-n-butylphosphine, triphenylphosphine, tri-o-tolylphosphine or triphenyl phosphite.

17. A process according to either of claims 1 or 2, wherein the catalyst is a mixture consisting of palladium acetate and triphenylphosphine or tri-o-tolylphosphine and, in particular diacetato-bis(tris-o-tolylphosphine)palladium(II).

18. A process according to either of claims 1 or 2, wherein the reaction is carried out in N,N-dimethylformamide as the solvent, the base is potassium acetate or sodium acetate, and the catalyst is a mixture consisting of palladium acetate and triphenylphosphine or tri-o-tolylphosphine and, in particular, diacetato-bis(tris-o-tolylphosphine)palladium(II).

**Revendications**

1. Procédé pour la préparation de composés de formule I

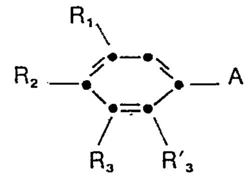 (I),

dans laquelle A est un radical alcényle en $C_{2-12}$, cycloalcényle en $C_{4-8}$ ou un groupe 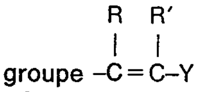
Y est –CN, –COR'', –COOH, –COOR'', $-CON(R'')_2$ ou

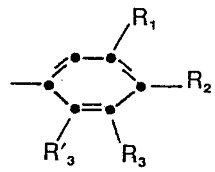 ,

R est l'hydrogène, le radical méthyle, –CN ou –COOR'',

R' est l'hydrogène, le radical méthyle ou $-CH_2COOR''$,

R'' est un radical alkyle en $C_{1-12}$ ou le radical phényle, et

$R_1$ et $R_3$, indépendamment l'un de l'autre, sont chacun l'hydrogène, le radical phényle, un radical alkyle en $C_{1-8}$, alcoxy en $C_{1-5}$, le radical $-CH(OCH_3)_2$, $-CH(OC_2H_5)_2$,

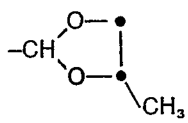 , 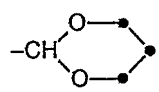 , 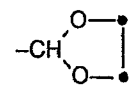 ,

–OH, Cl, F, $-NO_2$, –CN, –CHO, un radical –CO–(alkyle en $C_{1-4}$), –COO–(alkyle en $C_{1-4}$), –NHCO–(alkyle en $C_{1-4}$), le radical $-NH_2$, un radical –NH–(alkyle en $C_{1-4}$), –N(alkyle en $C_{1-4})_2$ ou le radical $-SO_3^-M^+$,

$R_2$ peut avoir la même signification que $R_1$ ou $R_3$, ou bien représente un groupe –CH = N-phényle ou $-C(R_4) = C(R_5)(R_6)$, et

$R'_3$ est l'hydrogène, le radical méthyle, –CN, –CHO ou $-SO_3C_6H_5$, ou bien $R_1$ et $R_2$ sont chacun l'hydrogène, et $R_3$ et $R'_3$ forment ensemble le radical –CH = CH–CH = CH–,

$R_4$ est l'hydrogène, un radical alkyle en $C_{1-4}$, le radical –CN ou un radical –COO–(alkyle en $C_{1-4}$),

$R_5$ est l'hydrogène, un radical alkyle en $C_{1-4}$, le radical –NHCHO, un radical $-(CH_2)_m$–COO–(alkyle en $C_{1-4}$) ou $-(CH_2)_m-CN$ avec m = 1 à 4,

$R_6$ est le radical –CN ou un radical –COO–(alkyle en $C_{1-4}$), et

$M^+$ est un cation métallique,

caractérisé en ce qu'on fait réagir un composé de formule II

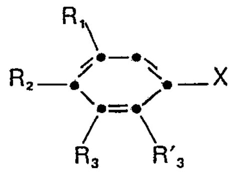

(II)

en présence d'un sel de métal alcalin ou de métal alcalino-terreux d'un acide monocarboxylique aliphatique ayant 1–12 atomes de carbone ou de l'acide benzoïque, d'un amide cyclique ou N,N-disubstitué servant de solvant, et, en tant que catalyseur, d'un composé du palladium contenant éventuellement de l'arsenic ou du phosphore, sur un composé de formule III

HA (III)

où les symboles A, Y, R, R', $R_1$, $R_2$, $R_3$ et $R'_3$ ont les significations données pour la formule I, X est l'iode ou le brome, et la teneur en palladium, rapportée au composé de formule II, est de 0,1 à 0,0001% molaire.

2. Procédé pour la préparation de composés de formule I

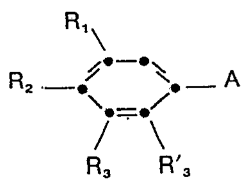

dans laquelle A est le groupe

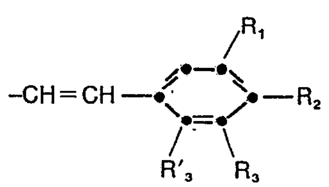

et

$R_1$ et $R_3$, indépendamment l'un de l'autre, sont chacun l'hydrogène, le radical phényle, un radical alkyle en $C_{1-8}$, alcoxy en $C_{1-5}$, le radical $-CH(OCH_3)_2$, $-CH(OC_2H_5)_2$,

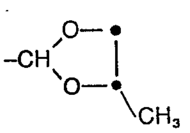 , 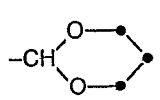 , 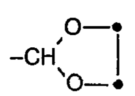 ,

–OH, Cl, F, $-NO_2$, –CN, –CHO, un radical –CO–(alkyle en $C_{1-4}$), –COO–(alkyle en $C_{1-4}$), –NHCO–(alkyle en $C_{1-4}$), le radical $-NH_2$, un radical –NH–(alkyle en $C_{1-4}$), –N(alkyle en $C_{1-4})_2$ ou le radical $-SO_3^-M^+$,

$R_2$ peut avoir la même signification que $R_1$ ou $R_3$, ou bien représente un groupe –CH = N–phényle ou $-C(R_4) = C(R_5)(R_6)$, et

$R'_3$ est l'hydrogène, le radical méthyle, –CN, –CHO ou $-SO_3C_6H_5$, ou bien $R_1$ et $R_2$ sont chacun l'hydrogène, et $R_3$ et $R'_3$ forment ensemble le radical –CH = CH–CH = CH–,

$R_4$ est l'hydrogène, un radical alkyle en $C_{1-4}$, le radical –CN ou un radical –COO–(alkyle en $C_{1-4}$)

$R_5$ est l'hydrogène, un radical alkyle en $C_{1-4}$, le radical –NHCHO, un radical $-(CH_2)_m$–COO–(alkyle en $C_{1-4}$) ou $-(CH_2)_m$–CN, avec m = 1 à 4,

$R_6$ est le radical –CN ou un radical –COO–(alkyle en $C_{1-4}$) et

$M^+$ est un cation métallique,

caractérisé en ce qu'on fait réagir sur de l'éthylène un composé de formule II

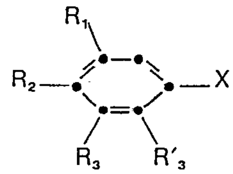

(II)

dans laquelle X est l'iode ou le brome, et $R_1$, $R_2$, $R_3$ et $R'_3$ ont les significations données ci-dessus, en présence d'un sel de métal alcalin ou de métal alcalino-terreux d'un acide monocarboxylique aliphatique ayant de 1 à 12 atomes de carbone ou de l'acide benzoïque, d'un amide cyclique ou N,N-disubstitué servant de solvant et, en tant que catalyseur, d'un composé du palladium contenant éventuellement de l'arsenic ou du phosphore, et sous une pression comprise entre 0,1 et 1 bar, la concentration du palladium, rapportée au composé de formule II, étant de 0,1 à 0,0001% molaire.

3. Procédé selon les revendications 1 et 2, caractérisé en ce que la concentration du palladium, rapportée au composé de formule II, est de 0,05 à 0,0001% molaire, en particulier de 0,01 à 0,001% molaire.

4. Procédé selon la revendication 1, caractérisé en ce qu'on utilise un composé de formule III dans laquelle A est le radical –C(R) = C(R')–Y, et au moins l'un des radicaux R et R' est l'hydrogène.

5. Procédé selon la revendication 1, caractérisé en ce qu'on utilise un composé de formule II dans laquelle X est le brome, $R'_3$ est l'hydrogène, $R_1$ et $R_3$, indépendamment l'un de l'autre, sont chacun l'hydrogène, le radical phényle, un radical alkyle en $C_{1-3}$, en particulier le radical méthyle, le radical méthoxy, $-NO_2$, –CN, –CHO, Cl, un radical –NHCO–(alkyle en $C_{1-2}$), –COO–(alkyle en $C_{1-2}$), –CO–(alkyle en $C_{1-2}$) ou –N(alkyle en $C_{1-2})_2$, et $R_2$ peut avoir la même signification que $R_1$ ou $R_3$ ou bien représente un groupe $-C(R_4) = C-(R_5)(R_6)$ où $R_4$ est l'hydrogène, le radical $-COOCH_3$ ou $-COOC_2H_5$, $R_5$ est l'hydrogène, le radical méthyle, éthyle, $-CH_2COOCH_3$, $-CH_2COOC_2H_5$ ou $-CH_2CH_2CN$, et $R_6$ est le radical –CN, $-COOCH_3$ ou $-COOC_2H_5$, l'un des deux radicaux $R_4$ et $R_5$ étant l'hydrogène.

6. Procédé selon la revendication 1, caractérisé en ce qu'on utilise un composé de formule II dans laquelle X est le brome, les radicaux $R_1$, $R_3$ et $R'_3$ sont chacun l'hydrogène, et $R_2$ est l'hydrogène, le radical phényle, méthyle, méthoxy, –CN, $-NO_2$, –CHO, Cl, $-NHCOCH_3$, $-N(CH_3)_2$ ou un groupe $-CH = CH-R_6$, et $R_6$ est le radical –CN, $-COOCH_3$ ou $-COOC_2H_5$.

7. Procédé selon la revendication 1, caractérisé en ce qu'on utilise un composé de formule III dans laquelle A est un radical alkylène ayant de 2–8 atomes de carbone, le radical cyclopentylène, cy-

clohexylène ou un groupe $-C(R)=C(R')-Y$, l'un des deux radicaux R et R′ est l'hydrogène et l'autre est le méthyle, ou bien les deux sont chacun l'hydrogène, et Y est le radical phényle, $-CN$ ou $-COOR''$, R″ étant le radical phényle ou un radical alkyle en $C_{1-4}$, en particulier le radical méthyle ou éthyle.

8. Procédé selon la revendication 1, caractérisé en ce qu'on utilise en tant que composé de formule III l'éthylène, l'acrylonitrile, ou l'acrylate de méthyle ou d'éthyle.

9. Procédé selon la revendication 2, caractérisé en ce qu'on prépare un composé de formule

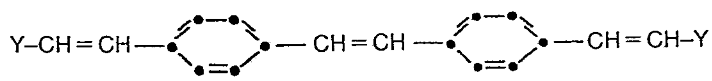

dans laquelle Y est le radical $-COOC_2H_5$, et en particulier $-CN$.

10. Procédé selon la revendication 1, caractérisé en ce qu'on prépare le 4-formylcinnamonitrile.

11. Procédé selon la revendication 2, caractérisé en ce qu'on prépare le 4,4′-stilbènedialdéhyde.

12. Procédé selon les revendications 1 et 2, caractérisé en ce qu'on utilise comme base un acétate, propionate ou benzoate d'un métal alcalin ou alcalino-terreux.

13. Procédé selon les revendications 1 et 2, caractérisé en ce qu'on utilise comme base l'acétate de potassium, et en particulier l'acétate de sodium.

14. Procédé selon les revendications 1 et 2, caractérisé en ce qu'on utilise comme amide un composé de formule IV

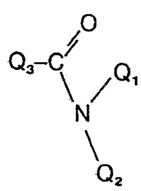

(IV)

dans laquelle $Q_1$ et $Q_2$, indépendamment l'un de l'autre, sont chacun un radical alkyle en $C_{1-8}$ à chaîne droite ou ramifiée, cycloalkyle en $C_{5-8}$ ou le radical benzyle, ou bien forment ensemble l'enchaînement $-(CH_2)_3-$, $-(CH_2)_4-$ ou $-(CH_2)_2-O-(CH_2)_2-$, et $Q_3$ est l'hydrogène, un radical alkyle en $C_{1-8}$ à chaîne droite ou ramifiée, ou forment avec $Q_1$ un enchaînement $-(CH_2)_q-$, avec $q = 3$, 4 ou 5.

15. Procédé selon les revendications 1 et 2, caractérisé en ce qu'on utilise comme solvant le N,N-diméthylformamide, le N,N-diéthylformamide, le N,N-diméthylacétamide, la N-méthylpyrrolidone ou l'hexaméthylphosphorotriamide.

16. Procédé selon les revendications 1 et 2, caractérisé en ce qu'on utilise comme catalyseur un mélange de $PdCl_2$, d'acétate de palladium ou de bis-(dibenzylidène-acétone)palladium(O) et de tri-n-butylphosphine, de tri-phénylphosphine, de tri-o-tolylphosphine ou de phosphite de triphényle.

17. Procédé selon les revendications 1 et 2, caractérisé en ce qu'on utilise comme catalyseur un mélange d'acétate de palladium et de triphénylphosphine ou de tri-o-tolylphosphine et en particulier le diacétato-bis-(tris-o-tolylphosphine)palladium(II).

18. Procédé selon les revendications 1 et 2, caractérisé en ce qu'on travaille dans du N,N-diméthylformamide en tant que solvant, et qu'on utilise en tant que base de l'acétate de potassium ou de sodium et, en tant que catalyseur, un mélange d'acétate de palladium et de tri-phénylphosphine ou de tri-o-tolylphosphine et en particulier le diacétato-bis-(tris-o-tolylphosphine)palladium(II).